# EUROPÄISCHE PATENTSCHRIFT

(11) **EP 0 982 039 B1**
(45) Veröffentlichungstag und Bekanntmachung des Hinweises auf die Patenterteilung: **20.04.2005**
(21) Anmeldenummer: 99250246.8
(22) Anmeldetag: 22.07.1999
(51) Int. Cl.: A61L 2/07, A61L 2/28, A61L 2/24

(54) **Dampfsterilisator**
Steam steriliser
Stérilisateur à vapeur

(30) Priorität: 20.08.1998 DE 19838678
(43) Veröffentlichungstag der Anmeldung: 01.03.2000
(73) Patentinhaber: Melag oHG, 10829 Berlin (DE)
(72) Erfinder: Kraftmeier, Jörg, 12051 Berlin (DE)
(74) Vertreter: Baumgärtel, Gunnar, Dr.

(56) Entgegenhaltungen:
- EP-A- 0 628 814
- EP-A- 0 808 631
- DE-U- 9 319 369

## Beschreibung

Die Erfindung betrifft einen Dampfsterilisator nach dem Oberbegriff des Anspruchs 1.

Zur Überprüfung der Erreichung der Scerilisationsbedingungen für eine ordnungsgemäße Sterilisation von Hohlkörpern, z. B. von hohlen medizinischen Instrumenten, sind schlauchartige Prüfkörper bekannt, die wendelförmig ausgebildet sind. Diese sind an einem Ende offen und am anderen Ende mit einem Indikatorhalter verbunden. In diesem ist ein chemischer Indikator angeordnet, der bei gleichmäßiger Dampfdurchdringung und Erreichen der Sterilisationsbedingungen, die die Luftentfernung aus dem zu sterilisierenden Produkt einschließt, seine Farbe verändert (Europanorm prEN 13060-2). Die Farbe ist mit der vom Hersteller des Indikatorsystems zur Verfügung gestellten Spezifikation für den Indikator zu vergleichen. Das Erreichen eines typischen Farbumschlages des Indikators gilt als Nachweis für das Erreichen der Sterilisationsbedingungen auch in dem zu sterilisierenden Hohlkörper.

Gemäß der Europanorm EN 554 vom November 1994, Punkt 6.3.4 muß ein Dampfdurchdringungstest zu Beginn eines jeden Tages durchgeführt werden, an dem der Sterilisator verwendet wird, wenn das Sterilisationsverfahren die Luftentfernung aus dem Produkt einschließt. Zu diesem Zweck wird der Prüfkörper in den leeren Nutzraum des Dampfsterilisators gelegt und der Sterilisationszyklus gestartet. Am Ende des Sterilisationszyklus ist der Prüfkörper aus dem Nutzraum zu entnehmen und der Farbumschlag in der oben angegebenen Weise zu prüfen.

Vor der Wiederverwendung des Prüfkörpers ist dieser auf Umgebungstemperatur abzukühlen und es ist sicherzustellen, daß die Innenteile trocken sind.

Die bisher verwendeten Prüfkörper werden lose in den Nutzraum eingelegt. Dadurch ergibt sich der Nachteil, daß der für das Sterilisiergut benötigte Platz eingeschränkt wird und das Prüfen des Farbumschlages erst nach dem Öffnen des Nutzraums möglich ist. Das ist besonders nachteilig, wenn nicht nur am Tagesbeginn eine Prüfung vorgenommen werden soll sondern, wie von Anwendern gefordert, die Sterilisationsbedingungen bei jedem Sterilisationsvorgang überwacht werden sollen. Es ist anders nicht möglich zu überprüfen, ob die Sterilisationsbedingungen bei jedem Vorgang auch wirklich erreicht wurden.

Es sind unterschiedliche Indikatoren bekannt, wobei diese sich nach Art, Größe und Ansprechverhalten unterscheiden. Deshalb ist es wichtig, daß der Indikator, der Prüfkörper, die Lage des Prüfkörpers im Sterilisator und der Sterilisator zusammen validiert und benutzt werden. Wenn ein Anwender unterschiedliche Sterilisatoren betreibt, kann es deshalb zu Verwechselungen der Prüfkörper und damit zu Fehlanzeigen kommen.

EP-A-0 628 814 beschreibt ein Prüfkörpersystem, das aus einer kombination von einem Indikatorhalter und einem direkt damit verbundenen hohlen Prüfkörper, der an das zu sterilisierende Gut angepaßt wird, besteht.

Aus der DE 43 19 398 C1 ist ein Indikatorträger bekannt, der fest mit dem Gehäuse verbunden ist. Dieser ist aber von außen nicht sichtbar, so daß auch bei diesem bei geschlossenem Dampfsterilisator und damit während des Sterilisationsvorganges die Sterilisationsbedingungen nicht geprüft werden können.

Der Erfindung liegt die Aufgabe zugrunde, bei der Sterilisation von Hohlkörpern in einem Dampfsterilisator auch bei geschlossenem Dampfsterilisator während jedes Sterilisationsvorganges die Sterilisationsbedingungen zu prüfen.

Erfindungsgemäß wird das gemäß den Merkmalen des Anspruchs 1 erreicht.

Bei einem Dampfsterilisator für die Sterilisation von Hohlkörpern, wobei zur Überprüfung der Sterilisationsbedingungen im Nutzraum des Dampfsterilisators mindestens ein hohler Prüfkörper verwendet wird, der an einem Ende offen ist, am anderen Ende mit einem Indikatorhalter für einen Indikator zum Nachweis des Erreichens der Sterilisationsbedingungen verbunden ist und mindestens die gleiche Länge wie der Hohlkörper aufweist, ist erfindungsgemäß der Indikatorhalter so am Gehäuse des Dampfsterilisators angebracht, daß der Indikator von außen sichtbar und/oder ohne durch die Tür des Dampfsterilisators greifen zu müssen, erreichbar ist und während des Betriebes des Dampfsterilisators auf der Temperatur des Dampfes im Nutzraum liegt, daß der sich an den Indikatorhalter anschließende Abschnitt des Prüfkörpers außerhalb der Aufnahmen für das zu sterilisierende Gut im Nutzraum des Dampfsterilisators angeordnet ist und daß der Prüfkörper während der Beaufschlagung mit Dampf so gesichert ist, daß er nicht geöffnet werden kann, um damit Verbrühungen zu verhindern.

Die Sicherung des Prüfkörpers gegen Öffnen während der Beaufschlagung mit Dampf kann in bekannter Weise erfolgen.

Eine solche Anordnung und Verbindung des Prüfkörpers mit dem Dampfsterilisator gewährleistet, daß jedem Sterilisator stets der passende Prüfkörper zugeordnet ist und Fehlmessungen wegen Verwechselungen und Lageänderungen der Prüfkörper ausgeschlossen sind.

Da der Indikator von außen sichtbar angebracht ist, kann während des Ablaufs des Sterilisationsvorganges bei geschlossenem Dampfsterilisator laufend überprüft werden, ob die Sterilisationsbedingungen erreicht sind. Falls noch Luft im Prüfkörper und damit auch in den zu sterilisierenden Hohlkörpern sein sollte, ist das an einem unvollständigen Farbumschlag erkennbar. In diesem Fall ist bereits während des Ablaufs der Sterilisation erkennbar, daß eine Störung am Dampfsterilisator vorliegt, die zu einer unzureichenden Sterilisation führen würde.

Nach Beendigung eines Sterilisationsvorganges läßt sich Restfeuchte aus dem Prüfkörper in einfacher Weise dadurch entfernen, daß der Prüfkörper nach Abnahme eines Verschlusses des Indikatorhalters mittels der Vakuumpumpe des Sterilisators durchgesaugt wird. Dadurch entfällt das aufwendigere Durchblasen mittels einer Druckluftquelle außerhalb des Sterilisators.

Um den Indikator mit der Temperatur des Dampfes im Nutzraum beaufschlagen zu können, ist der Indikatorhalter an der Vorderseite des Dampfsterilisators beheizbar angeordnet. Zusätzlich ist es zweckmäßig, den Indikatorhalter in einer Wärmeisolierung so anzuordnen, daß anfallendes Kondensat in den Nutzraum zurückfließen kann.

Die Beheizung erfolgt in einer Ausführungsform dadurch, daß der Indikatorhalter von Dampf umströmbar ist. In einer weiteren Ausführungsform ist der Indikatorhalter elektrisch beheizbar, indem er von einer Heizwendel umgeben ist, die exakt die Temperatur des Dampfes im Nutzraum reproduziert.

Es ist zweckmäßig, daß der Indikatorhalter oberhalb der Tür des Dampfsterilisators außerhalb des Nutzraumes angeordnet ist, da der Indikator dort besonders gut erkennbar und erreichbar ist.

In einer Ausführungsform erstreckt sich der Prüfkörper durch den Nutzraum und das offene Ende des Prüfkörpers liegt im unteren Bereich des Nutzraums.

In einer weiteren Ausführungsform ist der Prüfkörper im Bereich des Indikatorhalters angeordnet.

Es ist zweckmäßig, daß der Prüfkörper zumindest abschnittsweise wendelförmig ausgebildet ist, um einen Prüfkörper großer Länge auf kleinstem Raum unterbringen zu können.

Die Erfindung soll in Ausführungsbeispielen anhand von Zeichnungen erläutert werden. Es zeigen:
- Fig. 1: einen Schnitt durch einen Dampfsterilisator mit einem oberhalb der Tür angeordneten und durch Dampf beheizbaren Indikatorhalter;
- Fig. 2: einen Dampfsterilisator nach Fig. 1 mit einem elektrisch beheizbaren Indikatorhalter;
- Fig. 3: einen Dampfsterilisator nach Fig. 1 mit einem im Bereich des Indikatorhalters angeordneten Prüfkörper.

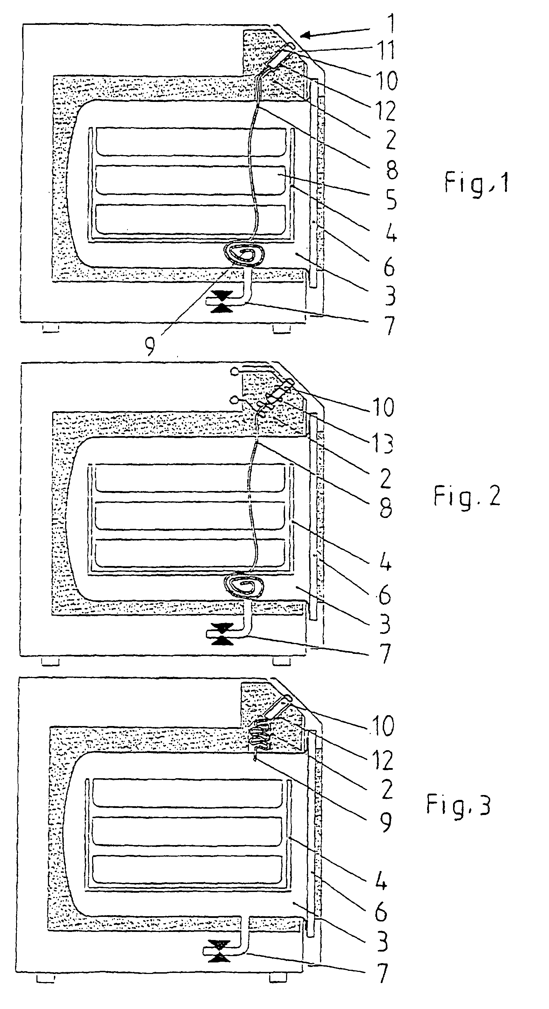

In der Fig. 1 weist ein Dampfsterilisator 1 wie üblich eine Wärmeisolierung 2 auf, die einen Nutzraum 3 für die Aufnahme eines Gestells 4 mit Tabletts 5 für die zu sterilisierenden Hohlkörper umschließt. An der Vorderseite ist eine isolierte Tür 6 für die Beschickung des Nutzraumes 3 vorgesehen. Der Dampfsterilisator 1 kann über eine Entlüftungsleitung 7 entlüftet werden.

Im Nutzraum 3 ist ein schlauchförmiger Prüfkörper 8 vorgesehen, der an einem Ende 9 offen ist, um das Entweichen von Luft und das Eindringen von Dampf zu ermöglichen. Das andere Ende des Prüfkörpers 8 wird durch einen Indikatorhalter 10 gebildet. Dieser ist in der Wärmeisolierung 2 angeordnet und ragt aus einer Frontplatte 11 nach außen, so daß der im Indikatorhalter 10 enthaltene Indikator (nicht dargestellt) von außen erkennbar und erreichbar ist. Der Indikatorhalter 10 ist in der Wärmeisolierung 2 von einem Dampfstutzen 12 umgeben, so daß er mittels Dampf beheizbar ist und damit auf der im Nutzraum 3 herrschenden Temperatur gehalten wird.

Der schlauchförmige Prüfkörper 8 verläuft bei der Ausführungsform der Fig. 1 neben dem Gestell 4 bis in den unteren Bereich des Nutzraums 3 und ist dort gewendelt.

Durch die erfindungsgemäße Anordnung des Prüfkörpers ist dieser fester Bestandteil des Dampfsterilisators, so daß er in seiner Ausführung auf dessen Besonderheiten abgestimmt werden kann und Fehlanzeigen des Indikators dadurch weitestgehend vermieden werden können.

Die Ausführungsform der Fig. 2 entspricht im wesentlichen der Ausführungsform der Fig. 1. Im Unterschied zu dieser wird aber der Indikatorhalter nicht mittels Dampf sondern mittels einer elektrischen Heizung 13 beheizt.

Auch die Ausführungsform der Fig. 3 entspricht im wesentlichen der Ausführungsform der Fig. 1. Im Unterscheid zu diesem erstreckt sich der Prüfkörper nicht durch den gesamten Nutzraum sondern ist komplett oberhalb des Gestells 4 in der Wärmeisolierung 2 angeordnet.

## Patentansprüche

1. Dampfsterilisator für die Sterilisation von Hohlkörpern, wobei zur Überprüfung der Sterilisationsbedingungen im Nutzraum des Dampfsterilisators mindestens ein hohler Prüfkörper verwendet wird, der an einem Ende offen ist, am anderen Ende mit einem Indikatorhalter für einen Indikator zum Nachweis des Erreichens der Sterilisationsbedingungen verbunden ist und mindestens die gleiche Länge wie der Hohlkörper aufweist,
**dadurch gekennzeichnet,**
**daß** der Indikatorhalter (10) beheizbar angeordnet ist und so ander Vorderseite des Gehäuses des Dampfsterilisators (1) angebracht ist, dass der Indikator von außen sichtbar und/oder ohne durch die Tür (6) des Dampfsterilisators greifen zu müssen, erreichbar ist und während des Betriebes des Dampfsterilisators (1) auf der Temperatur des Dampfes im Nutzraum (3) liegt, dass der sich an den Indikatorhalter (10) anschließende Abschnitt des Prüfkörpers (8) außerhalb der Aufnahmen (4, 5) für das zu sterilisierende Gut im Nutzraum (3) des Dampfsterilisators (1) angeordnet ist und daß der Prüfkörper (8) während der Beaufschlagung mit Dampf so gesichert ist, daß er nicht geöffnet werden kann.

2. Dampfsterilisator nach Anspruch 1, **dadurch gekennzeichnet, daß** der Indikatorhalter (10) in einer Wärmeisolierung (2) so angeordnet ist, daß anfallendes Kondensat in den Nutzraum (3) zurückfließen kann.

3. Dampfsterilisator nach mindestens einem der vorhergehenden Ansprüche, **dadurch gekennzeichnet, daß** der Indikatorhalter (10) von Dampf umströmbar ist.

4. Dampfsterilisator nach mindestens einem der vorhergehenden Ansprüche, **dadurch gekennzeichnet, daß** der Indikatorhalter (10) elektrisch beheizbar ist.

5. Dampfterilisator nach Anspruch 4, **dadurch gekennzeichnet, daß** der Indikatorhalter (10) von einer Heizwendel (13) umgeben ist.

6. Dampfsterilisator nach mindestens einem der vorhergehenden Ansprüche, **dadurch gekennzeichnet, daß** der Indikatorhalter (10) oberhalb der Tür (6) des Dampfsterilisators (1) außerhalb des Nutzraumes und von außen erreichbar angeordnet ist.

7. Dampfsterilisator nach mindestens einem der vorhergehenden Ansprüche, **dadurch gekennzeichnet, daß** sich der Prüfkörper (8) durch den Nutzraum (3) erstreckt und daß das offene Ende (9) des Prüfkörpers (8) im unteren Bereich des Nutzraums (3) liegt.

8. Dampfsterilisator nach mindestens einem der Ansprüche 1 bis 6, **dadurch gekennzeichnet, daß** der Prüfkörper (8) im Bereich des Indikatorhalters (10) angeordnet ist.

9. Dampfstrilisator nach mindestens einem der vorhergehenden Ansprüche, **dadurch gekennzeichnet, daß** der Prüfkörper (8) zumindest abschnittsweise wendelförmig ausgebildet ist.

## Claims

1. Steam sterilizer for the sterilization of hollow pieces, at least one hollow test piece being used in the working space of the steam sterilizer in order to check the sterilization conditions, which test piece is open at one end, is connected at the other end to an indicator holder for an indicator for verifying that the sterilization conditions have been achieved, and has at least. the same length as the hollow piece, **characterized in that** the indicator holder (10) is arranged in such a way that it can be heated and is attached to the front side of the housing of the steam sterilizer (1) in such a way that the indicator can be seen from outside and/or can be reached from outside without having to reach through the door (6) of the steam sterilizer and is at the temperature of the steam in the working space (3) during the operation of the steam sterilizer (1), **in that** that section of the test piece (8) which adjoins the indicator holder (10) is arranged outside the receptacles (4, 5) for the material to be sterilized in the working space (3) of the steam sterilizer (1), and **in that** the test piece (8), during the admission of steam, is secured in such a way that it cannot be opened.

2. Steam sterilizer according to Claim 1 **characterized in that** the indicator holder (10) is arranged in a heat insulation (2) in such a way that collecting condensate can flow back into the working space (3).

3. Steam sterilizer according to at least one of the preceding claims, **characterized in that** steam can flow around the indicator holder (10).

4. Steam sterilizer according to at least one of the preceding claims, **characterized in that** the indicator holder (10) can be heated electrically.

5. Steam sterilizer according to Claim 4, **characterized in that** the indicator holder (10) is surrounded by a heating coil (13).

6. Steam sterilizer according to at least one of the preceding claims, **characterized in that** the indicator holder (10) is arranged above the door (6) of the steam sterilizer (1) outside the working space and in such a way that it can be reached from outside.

7. Steam sterilizer according to at least one of the preceding claims, **characterized in that** the test piece (8) extends through the working space (3), and **in that** the open end (9) of the test piece (8) lies in the bottom region of the working space (3).

8. Steam sterilizer according to at least one of Claims 1 to 6, **characterized in that** the test piece (8) is arranged in the region of the indicator holder (10).

9. Steam sterilizer according to at least one of the preceding claims, **characterized in that** at least sections of the test piece (8) are of helical design.

## Revendications

1. Stérilisateur à vapeur destiné à la stérilisation de corps creux, au moins un échantillon creux étant utilisé pour vérifier les conditions de stérilisation dans l'enceinte de stérilisation du stérilisateur à vapeur, lequel échantillon est ouvert à une extrémité et est relié au niveau de l'autre extrémité à un support pour un indicateur destiné à démontrer que les conditions de stérilisation sont atteintes et lequel échantillon a au moins la même longueur que le corps creux, **caractérisé en ce que** le support d'indicateur (10) est agencé de manière à pouvoir être chauffé et est disposé sur la face avant du carter du stérilisateur à vapeur (1) de telle sorte que l'indicateur est visible de l'extérieur et/ou est accessible sans qu'il soit nécessaire de le saisir à travers la porte (6) du stérilisateur à vapeur (1) et, en cours de service du stérilisateur à vapeur (1), se situe à la température de la vapeur dans l'enceinte de stérilisation (3), **en ce que** la partie de l'échantillon (8) reliée au support d'indicateur (10) est située en dehors des logements (4, 5) destinés à recevoir les corps à stériliser dans l'enceinte de stérilisation (3) du stérilisateur à vapeur (1) et **en ce que**, pendant l'alimentation en vapeur, l'échantillon (8) est bloqué de telle sorte qu'il ne peut être ouvert.

2. Stérilisateur à vapeur selon la revendication 1, **caractérisé en ce que** le support d'indicateur (10) est agencé dans une isolation thermique (2) de telle sorte que le produit de condensation formé peut être réintroduit dans l'enceinte de stérilisation (3).

3. Stérilisateur à vapeur selon au moins une des revendications précédentes, **caractérisé en ce que** la vapeur circule tout autour du support d'indicateur (10).

4. Stérilisateur à vapeur selon au moins une des revendications précédentes, **caractérisé en ce que** le support d'indicateur (10) peut être chauffé électriquement.

5. Stérilisateur à vapeur selon la revendication 4, **caractérisé en ce que** le support d'indicateur (10) est entouré par un filament de chauffe (13).

6. Stérilisateur à vapeur selon au moins une des revendications précédentes, **caractérisé en ce que** le support d'indicateur (10) est agencé au-dessus de la porte (6) du stérilisateur à vapeur (1) à l'extérieur de l'enceinte de stérilisation (3) et accessible de l'extérieur.

7. Stérilisateur à vapeur selon au moins une des revendications précédentes, **caractérisé en ce que** l'échantillon (8) s'étend à travers l'enceinte de stérilisation (3) et **en ce que** l'extrémité ouverte (9) de l'échantillon (8) est disposée dans la partie inférieure de l'enceinte de stérilisation (3).

8. Stérilisateur à vapeur selon au moins une des revendications 1 à 6, **caractérisé en ce que** l'échantillon (8) est agencé dans la zone du support d'indicateur (10).

9. Stérilisateur à vapeur selon au moins une des revendications précédentes, **caractérisé en ce que** l'échantillon (8) est conçu au moins dans certaines zones sous forme de spirale.
